# EUROPEAN PATENT APPLICATION

(11) **EP 1 284 144 A1**
(43) Date of publication of application: **19.02.2003**
(21) Application number: 01402182.8
(22) Date of filing: 16.08.2001
(51) Int. Cl.: A61K 39/00, C12N 5/22, C12N 5/24, A61P 35/00

(54) **Anti-tumor vaccines**

(71) Applicant: Cellvax, 78400 Chatou (FR)
(72) Inventor: Wei, Ming Xing, 78400 Chatou (FR); Kopinski, Piotr Jan, 31-021 Krakow (PL)
(74) Representative: Texier, Christian

(57) **Abstract**

The invention relates to a method of preparing a cancer vaccine, wherein tumor cells are fused with either dendritic cells, or lymphocytes B or T, said cancer cells may be modified in order to decrease the expression of a major determinant of said cancer. The invention also relates to a vaccine composition comprising the hybridomas obtained with said method.

## Description

The present invention is drawn to a method of preparing a cancer vaccine, wherein tumor cells are fused with either dendritic cells, or lymphocytes B or T, said cancer cells may be modified in order to decrease the expression of a major determinant of said cancer. The invention is also drawn to a vaccine composition comprising the hybridomas obtained with said method.

Cancer cells escape control of cellular factors and proliferate without control, thereby initiating tumors. Furthermore, said cells may escape the site of the primary tumor and establish metastasis in other organs. Cancers are a major cause of mortality.

It is believed that cancer develop because, as cancer cells proliferate without control, they also are able to escape the immune system that does not recognize the tumor phenotype and does not destroy them.

One of the recent approach for curing cancer is to try to stimulate the immune system, in order to get it to fight and destroy the cancer cells. For this approach, people often rely on major determinant that are often over-expressed by the tumor cells. Such major determinants include PSA pour prostate cancer, IGF-1 for as many as 17 cancers among which breast, colon, brain (gliobastomas), liver, lung, pancreas cancers or teratocarcinomas, MUC-1, which is frequently expressed in breast cancer tissues, MART-1 / Melan A or gp100 in melanomas....

For example, one approach would use Antigen Presenting Cells (APC) such as dendritic cells, pulse them *in vitro* with peptides known to be presented by the MHC (preferably MHC-I), differentiate *in vitro* naive T cells to cytotoxic CD8 T cells (CTL) in presence of these APC, and reinject these CTL to the patient. This method would use autologous (issued from the patient) cells.

Nevertheless, this approach does not give as good results as expected, as the life time of the CTL may not always be long enough to completely reduce the tumor, and as tumor cells often under-express MHC I molecules, thereby reducing the efficacy of this method.

It is therefore important to design new methods for inducing an immunity against tumors, that would preferably be long lasting.

It is an aim of the present invention to disclose a method for preparing an anti tumor vaccine for use in a patient in need, comprising the step of fusing a tumor cell obtained from said patient to a lymphocyte B or a lymphocyte T obtained from said patient, in order to obtain an hybridoma.

The method of the invention in this first embodiment, is even bettered when said vaccine also comprises an hybridoma which is the product of fusion of a tumor cell and an activated dendritic cell from the patient.

It is preferred when said vaccine, in the method of the invention comprises hybridomas product of the fusion of tumor cells with lymphocytes B, T and activated dendritic cells.

In a specific embodiment, said tumor cell is transformed before fusion with an exogenous vector, whose expression product decreases the production of a major determinant of said tumor. As discussed above, "major determinants of tumors" is intended to mean proteins that are over-expressed in said tumors, or tumor markers. The literature describes such markers, and the list is not limited to but includes PSA, IGF-1, MUC-1, MART-1 / Melan A or gp100.

In another embodiment, the invention is drawn to a method for preparing an anti tumor vaccine for use in a patient in need, comprising the step of fusing a tumor cell obtained from said patient, wherein said tumor cell is transformed before fusion with an exogenous vector, whose expression product decreases the production of a major determinant of said tumor, to a cell obtained from said patient, chosen in the group consisting of an activated dendritic cell, a lymphocyte B and a lymphocyte T, in order to obtain an hybridoma.

The vaccine compositions obtained by said methods of the invention are also an object of the invention.

The invention is also drawn to a vaccine composition comprising an hybridoma product of the fusion between a cancer cell and a B or a T lymphocyte.

In a preferred embodiment, the vaccine composition of the invention also comprises an hybridoma which is the product of fusion of a tumor cell and an activated dendritic cell from the patient, and in the most preferred embodiment, the vaccine composition of the invention comprises hybridomas product of the fusion of tumor cells with lymphocytes B, T and activated dendritic cells.

The invention also covers a vaccine composition comprising an hybridoma product of the fusion between a tumor cell, wherein said tumor cell is transformed before fusion with an exogenous vector, whose expression product decreases the production of a major determinant of said tumor, and a cell chosen in the group consisting of an activated dendritic cell, a lymphocyte B and a lymphocyte T.

In another embodiment, the invention intends to cover a method for treating a cancer patient in need, comprising the steps of:
a) fusing cancer cells from said patient with B or T lymphocytes of said patients in order to form hybridomas,
b) injecting said hybridomas to said patient.

Alternatively, in a further embodiment, the invention also covers a method for treating a cancer patient in need, comprising the steps of:
a) fusing tumor cells obtained from said patient, wherein said tumor cells is transformed before fusion with an exogenous vector, whose expression product decreases the production of a major determinant of said tumor, to cells obtained from said patient, chosen in the group consisting of activated dendritic cells, lymphocytes B lymphocytes T, and mixtures thereof, in order to obtain an hybridoma,
b) injecting said hybridomas to said patient.

The invention also encompasses the use of an hybridoma product of the fusion between a tumor cell, wherein said tumor cell is transformed before fusion with an exogenous vector, whose expression product decreases the production of a major determinant of said tumor, and a cell chosen in the group consisting of an activated dendritic cell, a lymphocyte B and a lymphocyte T, for the preparation of a drug, in order to treat cancer.

The invention also encompasses the use of an hybridoma product of the fusion between a cancer cell and a B or a T lymphocyte, for the preparation of a drug, intended to treat cancer.

The use of the vaccines compositions according to the invention for the treatment of cancer is also within the scope of the invention.

The present invention indeed demonstrates that, in animal models, the injection of hybridomas obtained after fusion of cancer cells with dendritic cells, T lymphocytes, B lymphocytes, after reinjection, are able to reduce the tumor.

In the preferred embodiment of the invention, the tumor cells that are fused to either B or T lymphocytes or dendritic cells are transformed with a vector that expresses a product that is able to repress production of the major determinant of said tumor.

In one embodiment, the cancer is a cancer associated with surexpression of IGF-1, and is preferably chosen from the group consisting of breast, liver, lung, brain, colon and pancreas cancers. In particular said cancer is chosen from the group consisting of glioblastomas, prostate carcinomas, astrocytomas, meningiomas, sarcomas, thyroid adenomas, estradiol regulated and non regulated breast carcinomas, Wilms tumors, colon carcinomas, squamous cell carcinomas, small cell lung carcinomas, teratocarcinomas, and hepatocarcinomas.

In the preferred embodiment, said major tumor determinant the decrease in the production of which is sought is Insulin-like Growth Factor I (IGF-I),

In one embodiment of the invention, said product expressed by said vector is an antisense molecule.

In another embodiment, said product expressed by said vector acts in a triple-helix complex.

Antisense and triple-helix strategies bypass inherent limitations of functional studies which are dependent upon naturally mutated or artificially mutagenized cells (5,7,23). Antisense RNA binds to sense strand RNA or to DNA resulting in the formation of RNA-RNA or RNA-DNA hybrids with both the arrest of translation and nuclease degradation of template RNA, while the other oligonucleotide approach to interruption of gene expression uses triple-helix forming oligonucleotides (TFOs). The TFO block RNA polymerase transit by forming a triple-helical structures with complementary DNA.

While the first approach works mostly at the traductional level, the second one promises to provide a new class of sequence specific DNA-binding agents which can target malignancies at the transcriptional level (8,24,25,26). TFOs have also been used to form intermolecular triplex structure that blocks binding of transcription factors and supresses transcription of specific growth modulating genes such as epidermal growth factor or growth factor receptor (27), oncogene myc (28,29), HER2 (30,31), Ha-ras (32) or Ki-ras (33).

Another method to obtain decrease of production of the major tumor determinant is homologous recombination. This is obtained by transforming the tumor cell with a vector that harbors two sequences homologous to the sequences in the targeted gene (the major determinant gene), and flanking one or more selection marker. Homologous recombination (double cross-over event) will replace the targeted gene (or a part of it, for example an exon) by the marker gene. This technique is well known in the art, and different modifications may easily be added by a person skilled in the art (such as positive and negative markers...)

The vaccine obtained by the method of the invention is intended to be used after reinjection to the patient. Indeed, it is expected that the hybridomas produced by fusion of the tumor cells (transformed or not) with the blood cells will be immunogenic enough to stimulate the immune system to recognize the tumor.

One should note that the rate of fusion may be low and that it is possible for some tumor cells to still be present in the mixture that is injected to the patient. In order to reduce the risk of dissemination of tumor, it is preferable to irradiate the cells before injection (in order to kill them), their role being mainly to be vectors of tumor antigenic determinants and stimulators of the immune system. For example, one could use a dose of about 5000 rads of gamma rays, about an hour before injection. Other methods for killing cells without destroying their integrity may also be used.

In a specific embodiment, said hybridomas are treated with trypsin or EDTA before injection, to lift them from the cell culture plate. It is also possible to both irradiate the cells then treat them with a protease such as trypsin.

Alternatively, the exogenous vector may comprise a suicide gene, which codes for a product able to transform an inactive substance to a cytotoxic substance. One can cite genes of Herpes Simplex Virus -1 thymidine kinase 1, that can be used with ganciclovir or acyclovir, the DTA gene, coding for the A fragment of the diphtheria toxin, described in Yagi *et al*., (1990, PNAS, 87, 9918-22), that may be under the control of an inducible promoter. Other genes may be used by the person skilled in the art, for such a purpose, for example the cytochrome P450 2B1 gene or any other gene describe in US 5,688,773 (the content of which is incorporated herein by reference, with regards to the suicide genes), that may be used with cyclophosphamide or ifosphamide.

The TK-HSV-1 suicide gene may also be mentioned more especially. The viral TK enzyme displays markedly greater affinity compared to the cellular TK enzyme for certain nucleoside analogues (such as acyclovir or gancyclovir). It converts them to monophosphated molecules, which can themselves be converted by the cellular enzymes to nucleotide precursors, which are toxic. These nucleotide analogues can be incorporated in DNA molecules undergoing synthesis, hence chiefly in the DNA of cells in a state of replication. This incorporation enables dividing cells such as cancer cells to be preferentially destroyed.

Another embodiment of the invention may be to specifically purify the hybridomas obtained after fusion between the cancer cells and the blood cells. This implies another manipulation step, which may lead to a loss of cells, thereby reducing the actual efficiency of the vaccine, although it is speculated that the presence of only a very small number of hybridomas may be sufficient to prime the immune system and lead to destruction of the tumor.

In a preferred embodiment, the hybridomas are injected subcutaneously, intravenously, intradermally, or directly within the tumor. The subcutaneous or intravenous injections are preferred.

The vector that can be used to transform the tumor cells contains preferably a promoter, as well as appropriate regions for regulating transcription. It also needs to be maintained in the host cell, thereby comprising an origin of replication.

The person skilled in the art is aware of different types of vectors that may be used, and of the different techniques employed to make them enter the host cells (electroporation, lipofection...).

The vector to be used in the invention preferably contains at least one selectable marker gene that encodes a protein necessary for the survival and growth of the host cell in a selective culture medium. Typical selection marker genes encode proteins that either confer resistance to antibiotics such as kanamycin, hygromycon or neomycin. Indeed, a preferred selectable marker is the kanamycin resistance gene, as it allows growth both in procaryotic and eucaryotic (mammalian) cells.

In one embodiment of the invention, said vector is a viral vector.

Said virus may be of human or of non-human origin, as long as it possesses the capability to infect the cells of the patient. In particular, said virus is chosen from the group consisting of adenoviridiae, retroviridiae (oncovirinae such as RSV, spumavirinae, lentivirus), poxviridiae, herpesviridiae (HSV, EBV, CMV...), iridiovirus, hepadnavirus (hepatitis B virus), papoviridiae (SV40, papillomavirus), parvoviridiae (adeno-associated virus...), reoviridiae (reovirus, rotavirus), togaviridiae (arbovirus, alphavirus, flavivirus, rubivirus, pestivirus), coronaviriadiae, paramyxoviridae, orthomixoviridae, rhabdoviridae (rabies virus), bunyaviridae, arenaviridae, picornaviridae (enterovirus, Coxsackievirus, echovirus, rhinovirus, aphtovirus, cardiovirus, hepatitis A virus...), Modified Virus Ankara, and derived viruses thereof.

By derived viruses, it is intended to mean that the virus possesses modifications that adapt it to the human being (if it is a virus from a non-human origin that could not infect human cells without said modifications), and/or that reduce its potential or actual pathogenicity. In particular, it is best if the virus used for the gene transfer is defective for replication within the human body. This is an important safety concern, as the control of the expression of the functional gene may be a concern for the implementation of the method of the invention. One does not either whish to have a dissemination to other cells or to other people of the viral vector carrying the gene of therapeutic interest.

This is why the viral vector used in the method of the invention is preferably deficient for replication, and would therefore be prepared with the help of a auxiliary virus or in a complementary cell line, that would bring in *trans* the genetic material needed for the preparation of a sufficient viral titer.

Such defective viruses and appropriate cell lines are described in the art, for example in US Patent 6,133,028 that describes deficient adeno-associated viruses (AAV) and the associated complementation cell lines, and the content of which is herein incorporated by reference. Other suitable viruses are described for example in WO 00/34497. For adenoviruses or AAV, it may be interesting to delete the E1 and/or E4 regions.

In another embodiment, said vector may be chosen from the group consisting of episomal and integrative plasmids.

It is possible to introduce said vector within the cells by means of a synthetic carrier, which can be chosen from the group consisting of a cationic amphiphile, a cationic lipid, a cationic or neutral polymer, a protic polar compound such as propylene glycol, polyethylene glycol, glycerol, ethano, l-methyl-L-2-pyrrolidone or their derivatives, and an aprotic polar compound such as dimethyl sulfoxide (DMSO), diethyl sulfoxide, di-n-propyl sulfoxide, dimethyl sulfone, sulfolane, dimethylformamide, dimethylacetamide, tetramethylurea, acetonitrile or their derivatives. The person skilled in the art is aware of synthetic vectors that can be used and allow a high level of transfection, such as Lifofectine and Lipofectamine reagents available from Life Technologies (Bethesda, MD).

It is also possible to use electroporation or any other method to introduce the vector within the cells.

In an embodiment of the invention, the fusion between the tumor cells and the blood cells is performed using polyethylen glycol - PEG, according to the teachings of A. Robin, (Immunochemistry in Practice, Blackwell, NY, 1988), the technical content of which is incorporated herein by reference.

One may also use laser radiation, according to the teachings of Ohkohchi *et al*. (Lasers Surg Med. 2000;27(3):262-8), the content of which is incorporated herein by reference.

One can also use another technique to fuse the cells, such as electroporation, or any other technique that is known in the art.

The invention will better be understood with reference to the following examples, which do not restrict the scope of the invention.

### EXAMPLES

### Example 1: Cell culture.

Human primary glioma cell line (Trojan et al. (1996) Neurosci. Lett. 212, 9-12, Anthony et al. (1998) Adv. Exp. Med. Biol. 451, 27-34, the content of which is incorporated herein by reference, especially with regard to the description of this cell line), CNS-1 rat glioma cells and PCC3 embryona carcinoma cells were used.

Cells were cultured in DMEM+F 12 (v/v) (GIBCO-BRL) supplemented with 10 % FCS, 2mM glutamine, 100 U/ml penicillin and 100 microg/ml streptomycin, at 37° C and 5 % C02.

Hygromycin B (Boehringer Mannheim) at a concentration of 0,5 mg/ml was added 48 hours after transfection to select for transfected cells. For experiments using rat C6 glioma as positive control Kiess et al. (1989) Endocrinology 124 , 1727-1736), the concentration of hygromycin B was changed to 2 mg/ml after one weak and maintained with each change of fresh medium during the next 3-4 month. For experiments with human primary gliomas, the concentration of hygromycin for cell culture was determined as previously described (Anthony et al. (1998) Adv. Exp. Med. Biol. 451, 27-34). B-104 rat neuroblastoma and C1300 mouse neuroblastoma cell lines (obtained from ATCC) were used as a negative control (Trojan et al. (1992) Proc. Natl. Acad. Sci. USA 89 , 4874- 4878).

Primary cell cultures of human glioma were derived from tumors of 5 glioblastoma patients during surgical resection. Surgical sections approximately 3 X 3 mm X 1 - 2 cm in length were placed in DMEM containing high glucose concentration, 100 U/ml penicillin and 100 microg/ml streptomycin. Specimens were then transferred to phosphate buffered saline (PBS) containing no Ca2+ or Mg2+ and dissected into 1-2 mm fragments. PBS was changed to PBS containing 0,3 mg/ml collagenase and the tissue was then incubated x 20 min at 37° C with gentle agitation and centrifuged at 1500 rpm X 5 min. The pellet was resuspended in DMEM containing 20 % FCS supplemented with 2mM glutamine, 100 U/ml penicillin, 100 microg/ml streptomycin and 10 ng/ml EGF. Cell suspensions were adjusted to a concentration of 2 million cells / well in 6-well plates and incubated at 37° C and 5 % C02 in culture medium containing 10 ng/ml EGF (Sigma) (GIBCO). After two days, dead cells were removed and incubation was continued in DMEM containing 10% FCS, and no EGF, for three additional days. The medium was then changed to DMEM minus FCS and incubation was continued x 48 hours. Following this first week, cells were maintained in 5% FCS / DMEM / 10% C02 / 37° C until stable transfection was established (approximately 4 weeks).

### Example 2: Plasmids.

The episome based plasmid pMT-Anti IGF-I was constructed as previously described (Trojan et al. (1992) Proc. Natl. Acad. Sci. USA 89, 4874-4878, the content of which is incorporated herein by reference, especially with respect with the plasmid). The vector pMT-EP, under the control of the metallothionein, MT-I, inducible promotor was used as its base. The casette contains the Epstein-Barr Virus origin of replication and the gene encoding nuclear antigen I which together drive extrachromosomal replication. Down stream of the insertion site is a poly A termination signal followed by the hygromycin B and tetracyclin resistance genes.

The vector expressing IGF-I triple helix (pMT-AG triple helix) was constructed as previously described Shevelev et al. (1997) Cancer Gene Therapy 4, 105-112 the content of which is incorporated herein by reference, especially with respect with the plasmid). This cassette consists of a 25 bp DNA fragment cloned into the vector pMT-EP which transcribes a third RNA strand forming a triple helix structure within the target region of the human IGF-I gene, between its transcription and translation initiation sites. The vector pMT-EP with either the lac-Z reporter gene, or cDNA expressing IGFII antisense RNA as insert was used in control experiments (Trojan et al. (1994) Proc. Natl. Acad. Sci. USA. 91, 6088-6092).

Vectors encoding MHC-I or B-7 antisense cDNA were constructed using pMT-EP containing the neomycine (G418) resistance gene instead of the hygromycin B resistance gene, and the MHC-I or B7 insert in antisense orientation in place of the IGF-I gene sequence.

The details of the vector expressing mouse and rat MHC-I antisense are as follow : the insertion is a 300 bp fragment, which contains a MHC-I a3 domain from the Kb locus, cloned in antisense orientation into the polylinker (GenBank accession number is L 22338 ; MIC-I gene H-2T24 : the range is 278 bp starting from nucleotide 595 and finishing 872).

Concerning the vector expressing human MHC-I antisense, the insertion is a 300 bp fragment, which contains a MHC-I a3 domain, cloned in antisense orientation (GenBank accession number is X 03071; HLA-A2 gene (exon 4): the range is 276 bp). In the vector expressing mouse and rat B7 anti-sense, the insertion is a 960 bp fragment composed of fragments of B7-1 and B7-2, both in antisense orientation (GenBank accession numbers are X60958 and L25606 respectively ; the range is 353 bp for B7-1 and 560 bp for B7-2).

Concerning the vector expressing human B7-1 and B7-2 antisense, the insertion is 1500 bp in antisense orientation (GenBank accession number is NM 005191 ; the range is 1549 bp).

### Example 3: Transfection.

Cultures of cells, 60-80 % confluent, were transfected in 6-well plates utilizing a ratio of 1 microg plasmid DNA per 4.10⁵ cells. The FuGENE 6 Transfection Reagent (Boehringer Mannheim) was used according to the supplier's instructions. To determine the efficiency of transfection, the process was carried out using the pMT-EP construct containing lac-Z as a reporter gene. Cell cultures were washed in PBS and incubated at 37° C in the presence of the staining solution which contained 5mM K3Fe(CN)6, 2mM MgC12, 0,8 mg/ml X-gal made in PBS.

The selected IGF-I « antisense» or « triple helix » cell clones (expressing MHC-I and B7) were co-transfected with vectors either encoding MHC-I or B-7 antisense cDNA, in the presence of 0,4 mg/ml of G-418.

### Example 4: Northern blot.

Content of IGF-I antisense RNA was determined in 50 % confluent cell cultures. Cells were deprived of serum and cultured overnight in DMEM containing 0,1 % BSA; 58 microM ZnSO₄ (Sigma) was then added x 5 hours to induce the NUI promoter. The cells were then washed in PBS and lysed in guanidine isothiocyanate (Fluka), 25 mM Na-citrate, 100 mM beta-mercaptoethanol and 0,5 % sodium N-lauralsarcosine. The lysed cell suspensions were extracted with water saturated phenol and chloroform and precipitated with ethanol x 3. Aliquots (10 microg / well) were electrophoresed in 1% agarose - 1 S % formaldehyde in the presence of MOPS buffer and then blotted onto nitrocellulose filters. Labeling of rat and human IGF-I cDNA and chicken beta actin cDNA and hybridizations were done according to methods known in the art; The Northern blot was used also to verify expression of IGFI in solid glioblastomas.

### Example 5: Immunocytochemistry and Flow cytometry analysis (FACS).

Following induction of the MTI promoter, cells were fixed in 4% paraformaldehyde. Immunocytochemical localization of IGF-I protein was done by the immunoperoxidase technique (Vectastain ABC kit, Vector Laboratories, Burlingame, CA, USA). Polyclonal antibodies against rat IGF-I and against human IGF-I were purchased from Valbiotech (Paris, France). Antibodies to mouse, rat and human ICAM-1, ICAM-2 and VCAM-1 were from PharMingen, San Diego, CA.

For FACS, paraformaldehyde-fixed cells were incubated one hour at 4°C with either monoclonal antibodies (mAb) to rat monomorphic MHC-I (Ox-18 from Valbiotech, Paris, France) or to human monomorphic MIC-I (W6/32 from ATCC, Bethesda, MD), or with a fusion protein CTLA4-lg to stain B-7 antigen. The cells were then washed and incubated 1 hour with FITC conjugated goat F (ab') 2 antimouse Ig (in the case of mAb anti-rat MHC) or anti human Ig (in the case of CTLA4-1g). Stained cells were analyzed in a FACSCAN flow cytometer (Becton Dickinson). The cell aliquots used for FACS were separated into two groups : one group of cells was non irradiated ; another group of human or rat glioma cells was irradiated with 5000 rads of 60 CO.

### Example 6: Fluorescein cell-death detection and electron microscopy

Apoptosis was determined by dUTP-fluorescein terminal transferase-labeling of nicked DNA (TUNEL apoptosis assay). The « In situ Cell Death Detection Kit, fluorescein » (Boehringer Mannheim) was used according to supplier's instructions.

Cells were fixed in 1 % glutaraldehyde and 4% formaldehyde in 0,1 M phosphate buffer, pH 7,2 ; rinsed x 40 min in 0,1 M phosphate buffer (3X) and then fixed again in phosphate - buffered 1% Os04 x 1 hour. After rinsing in distilled water for 20 min, the cells were stained with 2% uranyl acetate x 40 min at 60°C. They were then rinsed in distilled water (3X), dehydrated in progressive concentration of ethanol and 100% propylene oxide and embedded in Spurr's resin. 80-nm sections were cut, placed on 200 mesh copper grids, and contrasted with lead citrate and uranyl acetate. Photomicrographs were taken using Philips EM-2000 electron microscope operating at 80 kV.

### Example 7: Ex vivo generation of dendritic cells, lymphocytes B and T cells from freshly drawn blood

Different techniques of generation of dendritic cells lymphocytes B and T cells were compared
1) Using MACS CD34 Cell Isolation Kit, CD34+ hematopoietic progenitor cells were isolated. Functional DC cells were generated by culturing CD34+ cells in the présence of GM-CSF, TNFalpha and SCF for 10 days (Siena et al. Exp ; Hemat. 23, 1465-1473, 1995). Thus 200 000 CD34 cells give rise to 6 - 8 000 000 activated DCs.
2) Using MACS CD14 MicroBeads, monocytes were isolated. Monocytes cultured in the presence of GM-CSF and IL-4 generated activated DCs (Pickl et al. J. Immunnol. 157, 3850-3859, 1996).
3) By stimulating the blood cells with combination of IL-4 (25 ng/ml) and GM-CSF (120 ng /ml) for 10 - 12 days in vitro.
4) Using microbeads labelled with antobodies CD 19 and CD20 for B cells or CD3 and CD7 for T cells (Dynal, France).

### Example 8: Fusion of dendritic cells, lymphocytes B and T cells with tumor transfected cells.

Activated DCs, lymphocytes B and T, obtained using one of the techniques mentioned in example 8 were fused with tumor IGF-I triple-helix transfected cells using polyethylen glycol - PEG according to the method described by Robin (Immunochemistry in Practice, Blackwell, NY, 1988). The activated dendritic cells, lymphocytes B and T cells (DCBT), were used with IGF-I triple-helix transfected cells at a ratio of 100 DCBT to 20 tumor transfected cells. (Comparatively the hybridomas of « antisense» type were prepared).

Fusion was carried out with 40% PEG in PBS without Ca⁺⁺and Mg⁺⁺.

### Example 9: In vivo experiment

5 x 10⁶ PCC3 cells were injected subcutaneously into 129 SV mice (Trojan et al. (1994) Proc. Natl. Acad. Sci. USA. 91, 6088-6092), and 0.5 x 10⁵ of CNS-1 cells into Lewis rats. In seven week old male Lewis rats, placed in a stereotactic frame, the brain was punctured by a syringe (2 mmm posterior and 4 mm laterla to bregma) to inject cells in 10 µl of medium. As a control, PBS solution was injected.

The following groups of animals, Lewis and 129 Sv, were considered (6 animals per group) according to type of injected cells :
a) parental cells;
b) IGF-I "triple-helix" transfected cells (TH IGF);
c) IGF-I "antisense" transfected cells (AS IGF);
d) IGF-I "triple-helix" transfected cells co-transfected with MHC-I and B7 antisense vector (TH IGF/ AS MHC /AS B7);
e) IGF-I "antisense" transfected cells cotransfected with MHC-I and B7 antisense vector (AS IGF /AS MEC /AS B7);
f) cell hybridomas composed of DCBT and cancer cells (DCBT/CC);
g) mixed cultures of DCBT and cancer cells transfected with IGF-I antisense or triple-helix type (DCBT/TH or DCBT/AS).

### RESULTS

### Immunocytochemical and Molecular characterization of tumoral cells

Human glioma cells established from five glioblastoma multiforme cancers demonstrated morphological characteristics similar to those previously described (Trojan et al. (1996) Neurosci. Lett. 212, 9-12). The percent of IGF-I positive cells in the human cell lines ranged from 50 to 70 %.Each cell line was subcloned to obtain IGF-I positive clones, and one cell line (HG1) was selected for further characterization.

All five cell lines derived from five human gliomas were successfully transfected using FuGENE technique.

IGF-I transfected cells derived from HG 1 line, antisens (pMT-Anti IGF-I) as well as IGF-I triple helix transfected cells (pMT-AG-Triple helix) did not stain immunocytochemically for IGF-I.

Comparatively labelled rat CNS-1 glioma and mouse PCC3 cells gave positve reaction, while these cells transfected with the vector coding either for the antisense or the triple helix were negatively stained.

The DCBT/CC hybridomas gave weak reaction with antibodies to IGF-I as compared to parental cells.

As expected DCBT/TH or DCBT/AS hybridomas were negative for IGF-I.

As far as other antigens were labelled using immunocytochemical staining of MHC-I, MHC-II, ICAM-1 and -2, and B7, the most important observation concerned the increase level of MHC-I and MHC-II, and especially of B7 presence in DCBT/TH and DCBT/AS « vaccines» (Table 1).

The frequency of transfection for the pMT-EP- LacZ vector, as determined by staining with X-gal at 48 hours after transfection, was in between 10 - 15 % for both rat and human glioma lines. No difference in transfection efficiency was observed for constructs containing the promoters, MT, CMV or HS.

Analysis of RNA of human glioma cells four weeks after transfection compared to RNA of parental cells was performed by Northern blot.

The RNA of non-transfected cells harbors two bands (7.5 kb and 1.0 kb) corresponding to the different processing stages of endogenous human IGF-I RNA.

The RNA of pMT-Anti IGF-I (antisense) transfected cells showed only an abundant 1.0 kb band, while RNA of pMT-AG triple helix transfected cells does not show any band when human IGF-I cDNA is used as probe, while Northern blot pattern of rat glioma cells were as previously published (Trojan et al. (1992) Proc. Natl. Acad. Sci. USA 89 , 4874-4878).

The expression of MHC-1 and B-7 molecules on the surface of transfected and parental human glioma cells was determined by flow cytometry using monoclonal antibody against the corresponding molecules.

Transfection with either the pMT-Anti IGF-I or pMT-AG triple helix vector induced increases of 13 - 15 % and 23 - 25 % in the expression of MHC-I and B-7, respectively.

Transfection with pMT-EP expressing the lac Z reporter gene or with vector expressing IGF-11 in place of IGF-I antisense RNA produced no increase in expression MHC-I or B-7 molecules.

The IGF-I antisense or triple-helix transfected cells did not express the MHC-I and B7 molecules above the parental cell background levels when co-transfected with vectors encoding MHC-I or B-7 antisense cDNA.

The rat glioma cells studied comparatively showed parallel results. The cells transfected with a vector expressing IGF-II in place of IGF-I antisense RNA a vector expressing the lac-Z reporter gene stained positively for IGF-I.

As far as MHC-I and B7 expression is concerned no difference between irradiated and non irradiated cells was observed.

**Table 1:**

| Immunocytochemical stainig of IGF-I and of immunogenic antigens in rat (CNS-1) and human (HG1) glioma, and mouse embryonal carcinoma (PCC3) cells. | | | | | | | |
|---|---|---|---|---|---|---|---|
| Cells | IGF-1 | ICAM-1 | ICAM-2 | VCAM-1 | CMH-I | CMH-II | B7 |
| HG1 | +++ | - | - | - | - | - | - |
| CNS-1 | +++ | - | - | - | - | - | - |
| PCC3 | ++ | - | - | - | - | - | - |
| | | | | | | | |
| HG1 IGF-I AS | - | + | +_ | +_ | ++ | - | ++ |
| CNS-1 IGF-1 AS | - | + | + | +_ | ++ | - | ++ |
| PCC3 IGF-I AS | - | + | + | + | ++ | - | + |
| | | | | | | | |
| HGI IGF-I TH | - | +_ | +_ | +_ | ++ | - | ++ |
| CNS-1 IGF-1 TH | - | +_ | + | +_ | ++ | - | ++ |
| PCC3 IGF-I TH | - | + | + | + | ++ | - | + |
| | | | | | | | |
| CNS-1 | | | | | | | |
| DCBT/CC | + | + | + | + | ++ | + | - |
| DCBT/IGF-ITH | - | + | + | + | ++++ | +++ | +++ |
| DCBTIIGF-1 AS | - | + | + | + | ++++ | +++ | +++ |
| | | | | | | | |
| PCC3 | | | | | | | |
| DCBT/CC | + | + | + | + | ++ | + | _ |
| DCBT/IGF-1 TH | - | + | + | + | ++++ | +++ | ++ |
| DCBT/IGF-1 AS | - | + | + | + | ++++ | +++ | +++ |

### Detection of apoptotic changes in IGF-I down-regulated cells

Apoptosis - induced morphological changes in pMT-Anti IGF-I and pMT-AG triple helix transfected cells were detected using the May-Grünwald-Giemsa technique and the TUNEL assay as described in the examples.

Individual apoptotic bodies and rosettes formed by smaller apoptotic elements surrounding the nucleus were observed. Earlier changes indicative of apoptosis in transfected cells were demonstrated by the TUNEL assay. Incorporation of fluorescein-conjugated dUTP was detected in approximately 60-70 % of the IGF-I antisense and triple helix transfected cells. In cultures of both IGF-I antisense and triple helix transfected cells, approximately 40-50 % of the cells became rounded and detached. These cells were all apoptotic.

In contrast, evidence of apoptosis was observed in less than 5-10 % of non-transfected cells.

The IGF-I « antisense » or « triple-helix » cell cultures co-transfected with
a) either MHC- or B-7 antisense expressing vectors showed also 60-70 % of apoptotic cells;
b) with both anti - MHC-I and anti - B-7 vectors, showed the diminished number, 20-30 %, of apoptotic cells.

These results are in agreement with electron micrograph images of IGF-I «antisense» and « triple-helix» transfected cells and with cells co-transfected with MHC-I and/or « antisense» vectors.

The apoptotic changes occurred within 5-6 hours after incubation of transfected cells in the présence of 58 µM of ZnSO₄.

As far as cell hybridomas are concerned, the DCBT/CC cultures showed only 5 - 8 % of apoptotic cells.

On the contrary the DCBT/TH or DCBT/AS cultures showed apoptotic phenomenon in about 50 % of cell population.

### In vivo experiment

When 5 x 10⁶ PCC3 cells were injected subcutaneously into 129 SV mice, tumors appeared alter 20-24 days (21 days tumor is 5 mm in diameter).

No tumors were observed in animals injected with cells that were stable transfected with IGF-I « triple-helix » vector, and expressing MHC-I and B-7. This observation was made both in 129 Sv mice and in Lewis rats. The latest animals remained tumor free for 8-10 months.

In contrast, the injection of IGF-I « triple-helix» transfected cells co-transfected with IVMC-I and B7 antisense vector was associated with development of tumor in six out of six animals. The efficiency of triple-helix approach to suppress PCC3 mouse teratocarcinoma or CNS-1 rat glioma established tumors were 83 and 67 %, respectively.

The injection of TH IGF cells, suppressed the established teratocarcinoma tumors alter 5 - 7 weeks, and glioma tumors after 4 - 5 weeks.

*In vivo* experiments using IGF-1 « antisense » vector in place of the IGF-I «triple-helix» vector gave similar results.

Injection of activated «dendritic cells, lymphocytes B and T cells / cancer cells» hybridomas (DCBT/CC) derived either from PCC 3 or CNS-1 was investigated.

Injection of DCBT/CC into bearing tumors animals suppressed the established tumors in 65 % (CNS-1) and 75 % (Pcc3) of the animals.

The experience was repeated using cell hybridomas composed of IGF-I triple-helix cells and activated dendritic cells and B and T lymphocytes (DCBT/TH). In this case, the subcutaneous injection of DCBT/TH into bearing glioma animals completely suppressed the tumors in 85-90 % (CNS-1) and 95-100 % (PCC3) of the animals after about 3 weeks (Table 2).

Thus, the result presented in the present application demonstrate that:
- use of a repressor of production of IGF-I in cancer cells increases the production of MHC-I and B7 molecules (involved in immunomodulation), thereby being favorable for stimulating the immune system, and also increases apoptosis of these cells,
- fusion of cancer cells with blood cells (activated dendritic cells, T lymphocytes, B lymphocytes) leads to a regression of the tumor,
- said regression being better and faster when the hybridomas are produced with cancer cells in which IGF-1 expression is repressed.

**Table 2.**

| Suppression of tumors expressing IGF-I using different strategies of « anti-gene » anti IGF-I cellular therapy . Comparison of CNS-1 glioma with other tumors expressing IGF-I *. | | | | | |
|---|---|---|---|---|---|
| tumor | glioma | glioma | Terato -carcinoma | hepatoma | hepatoma |
| cells | C6 | CNS-1 | PCC3 | LFC | ATM |
| model | syngeneic | syngeneic | syngeneic | syngeneic | Transgenic mice |
| | DB IX | Levis | 129 SV | Wistar Commentry | ATIIIH/B6 |
| AS-IGF-I | 100% ** 4-5w*** | 50% 4-5w**** | 67% 5-7w**** | 90% 4-5w | 100% 3-4m***** |
| Th-IGF-I | | 67% 4-5 w | 83% 5-7 w | | 100% 4m |
| DCBT/CC | | 65 % 4-5w | 75 % 5-7w | | |
| DCBT/AS IGF-1 | | 70-80% 2-4w | 87-92% 4-6w | | |
| DCBT/TH IGF-I | | 85-90% 2-3w | 95-100% 3-4w | | |

| | | | | | |
|---|---|---|---|---|---|
| * (1993) Science 259 ; 94-7. (syngeneic model up to 1995) (1994) Proc. Natl. Acad. Sci. USA. 91 ; 6088-6092. (1997) Cancer Gene Ther. 4 ; 105-112. (1997) Cancer Gene Ther. 4 ; 276-285. (1998) Adv. Exp. Med. Biol. 451 ; 35-42. (2000) Life Sciences 68 ; 307-309. | | | | | |
| ** % of animals with total regression of tumors ; | | | | | |
| *** time of regression; | | | | | |
| **** six animals per group ; | | | | | |
| ***** supplement survival months of mice ATIIIH (generally dead at 6 months). | | | | | |

## Claims

1. A method for preparing an anti tumor vaccine for use in a patient in need, comprising the step of fusing a tumor cell obtained from said patient to a lymphocyte B or a lymphocyte T obtained from said patient, in order to obtain an hybridoma.

2. The method of claim 1, wherein said vaccine also comprises an hybridoma which is the product of fusion of a tumor cell and an activated dendritic cell from the patient.

3. The method of any of claims 1 or 2, wherein said vaccine comprises hybridomas product of the fusion of tumor cells with lymphocytes B, T and activated dendritic cells.

4. The method of any of claims 1 to 3, wherein said tumor cell is transformed before fusion with an exogenous homologous recombination vector, which targets a major determinant of said tumor, leading to inactivation of the gene of said major determinant.

5. The method of any of claims 1 to 3, wherein said tumor cell is transformed before fusion with an exogenous vector, whose expression product decreases the production of a major determinant of said tumor.

6. The method of claim 5, wherein said expression product is an antisense molecule.

7. The method of claim 5, wherein said expression product acts in a triple-helix complex.

8. The method of any of claims 4 to 7, wherein said vector also comprises a suicide gene.

9. The method of any of claims 5 to 8, wherein said vector is chosen from the group consisting of episomal and integrative plasmids.

10. The method of claim 9, wherein said transformation before fusion is performed by a method chosen from the group consisting of electroporation and lipofection.

11. The method of any of claims 4 to 8, wherein said vector is a viral vector.

12. The method of any of claims 1 to 11, wherein said fusion is performed using PEG.

13. The method of any of claims 1 to 12, wherein said cancer is a cancer associated with surexpression of IGF-1.

14. The method of claim 13, wherein said cancer is chosen from the group consisting of glioblastomas, prostate carcinomas, astrocytomas, meningiomas, sarcomas, thyroid adenomas, estradiol regulated and non regulated breast carcinomas, Wilms tumors, colon carcinomas, squamous cell carcinomas, small cell lung carcinomas, teratocarcinomas, and hepatocarcinomas.

15. The method of any of claims 4 to 14, wherein said major determinant is IGF-1.

16. A vaccine composition comprising an hybridoma product of the fusion between a cancer cell and a B or a T lymphocyte.

17. The vaccine composition of claim 16, further comprising an hybridoma product of the fusion between a cancer cell and an acitvated dendritic cell.

18. A method for preparing an anti tumor vaccine for use in a patient in need, comprising the step of fusing a tumor cell obtained from said patient, wherein said tumor cell is transformed before fusion with an exogenous vector, whose expression product decreases the production of a major determinant of said tumor, to a cell obtained from said patient, chosen in the group consisting of an activated dendritic cell, a lymphocyte B and a lymphocyte T, in order to obtain an hybridoma.

19. A vaccine composition comprising an hybridoma product of the fusion between a tumor cell, wherein said tumor cell is transformed before fusion with an exogenous vector, whose expression product decreases the production of a major determinant of said tumor, and a cell chosen in the group consisting of an activated dendritic cell, a lymphocyte B and a lymphocyte T.

20. The vaccine composition of any of claims 16, 17 or 19, as a medicament.

21. Use of an hybridoma product of the fusion between a tumor cell, wherein said tumor cell is transformed before fusion with an exogenous vector, whose expression product decreases the production of a major determinant of said tumor, and a cell chosen in the group consisting of an activated dendritic cell, a lymphocyte B and a lymphocyte T, for the preparation of a medicament in order to treat cancer.

22. Use of an hybridoma product of the fusion between a cancer cell and a B or a T lymphocyte, for the preparation of a medicament, intended to treat cancer.
